# EUROPEAN PATENT SPECIFICATION

(11) **EP 2 004 138 B1**
(45) Date of publication and mention of the grant of the patent: **20.11.2013**
(21) Application number: 06726403.6
(22) Date of filing: 16.03.2006
(51) Int. Cl.: A61K 9/00, A61K 47/36, A61P 3/04, A61P 3/06, A61P 3/08

(54) **GASTRIC RAFT COMPOSITION COMPRISING PROCESSED STARCHES FOR INDUCING SATIETY**
MAGEN-RAFT-ZUSAMMENSETZUNG MIT AUFBEREITETEN STÄRKEN ZUR AUSLÖSUNG EINES SÄTTIGUNGSGEFÜHLS
COMPOSITION DE BARRIÈRE GASTRIQUE COMPRENANT DES AMIDONS TRANSFORMÉS POUR INDUIRE LA SATIÉTÉ

(43) Date of publication of application: 24.12.2008
(73) Proprietor: Glycologic Limited, Glasgow G4 0BA (GB)
(72) Inventor: TESTER, Richard, Glasgow G4 0BA (GB); HOOPER, Denise, Glasgow G4 0BA (GB)
(74) Representative: Docherty, Robert Charles
(86) International application number: PCT/GB2006/000953
(87) International publication number: WO 2007/104905

(56) References cited:
- WO-A-03/051329
- US-A- 4 812 315
- MANDEL K G ET AL: "Review article: Alginate-raft formulations in the treatment of heartburn and acid reflux" ALIMENTARY PHARMACOLOGY AND THERAPEUTICS, vol. 14, no. 6, June 2000 (2000-06), pages 669-690, XP002402797 ISSN: 0269-2813
- WATERHOUSE ESTHER T ET AL: "An investigation into the efficacy of the pectin based anti-reflux formulation-Aflurax" INTERNATIONAL JOURNAL OF PHARMACEUTICS (KIDLINGTON), vol. 209, no. 1-2, 19 November 2000 (2000-11-19), pages 79-85, XP002402798 ISSN: 0378-5173
- OHTA ATSUTANE ET AL: "The alginate reduce the postprandial glycaemic response by forming a gel with dietary calcium in the stomach of the rat" INTERNATIONAL JOURNAL FOR VITAMIN AND NUTRITION RESEARCH, vol. 67, no. 1, 1997, pages 55-61, XP009073590 ISSN: 0300-9831
- DI LORENZO C ET AL: "PECTIN DELAYS GASTRIC EMPTYING AND INCREASES SATIETY IN OBESE SUBJECTS" GASTROENTEROLOGY, vol. 95, no. 5, 1988, pages 1211-1215, XP009073593 ISSN: 0016-5085
- ARORA S ET AL: "Floating drug delivery systems: A review" AAPS PHARMSCITECH 19 OCT 2005 UNITED STATES, vol. 6, no. 3, 19 October 2005 (2005-10-19), page 31p, XP009073596 ISSN: 1530-9932

## Description

### Field of the Invention

The present invention relates to gastric raft compositions. In particular it relates to gastric raft compositions for use in inducing satiety in an individual. It also provides a method of prolonging retention of a source of dietary energy in the stomach and methods of treatment of diseases characterised by hypoglycaemia, such as glycogen storage disease (GSD), and diseases such as diabetes.

### Background to the Invention

Obesity is an increasing global health problem and has been associated as a major cause and/or risk factor for many health problems such as cardiovascular diseases such as hypertension and arteriosclerosis, non-insulin dependent diabetes mellitus, osteoarthritis and certain cancers. In order to assist individuals with losing weight, many dietary aids and treatments have been proposed, including pharmaceutical, dietary or surgical interventions.

One approach which has been used is the use of food stuffs which induce a feeling of "fullness" in an individual in order to suppress hunger and thus reduce food intake. For example, it is well known and understood that polysaccharides provide bulk and satiety in the diet. In this context, the polysaccharide is a 'dietary fibre', which provides calorie free bulk in the diet. This may be defined as follows (http://www.pauls.com.au/information):

Dietary Fibre (or roughage) comprises the indigestible material from plants and includes any plant polysaccharide for which humans have no appropriate enzyme to break down. Dietary fibre provides indigestible bulk which encourages the normal elimination of body wastes. A high fibre content also makes foods more filling or satisfying.

Among the polysaccharides which are used as bulking agents in foods are alginates and pectins. Alginates are extracted from seaweed and are indigestible by the human digestive tract. The use of alginates as texturing aids in foods have been discussed elsewhere together with the associated chemistry (http://www.ispcorp.com). According to Us patent application 20050170059, alginates can be fed in food and drink products to provide bulk in the stomach and thus provide satiety by this bulking effect.

The actual use of alginates in solution to provide satiety (as dietary fibre) with associated transit times have been discussed and reviewed elsewhere (Hoad et al, 2004). Apart from the sensation of a full stomach (satiety), alginates and pectins can also contribute to satiety by being retained in the stomach longer than other components of foods. This property of alginates and pectins is apparently unusual given that they are sources of fibre. These features have been discussed elsewhere (Di Lorenzo et al., 1988; Torsdottir et al., 1991; Tiwary et al., 1997). Compared to methyl cellulose (MC) added to foods, gastric retention is longer with pectin - t½ of 116 versus t½ of 71 minutes for pectin and MC respectively for obese subjects (Di Lorenzo et al., 1988). Further examples of the use of polysaccharides for inducing satisfy are disclosed in WO2003/051329 and US 4 812 315.

The use of foodstuffs and formulations comprising a high dietary fibre concentration is associated with a number of potential side effects. A particular problem associated with reliance of such bulking agents to induce satiety and thus reduce overall calorie intake is, that sometimes, the consumption of too much dietary fibre causes colonic disturbance - in part due to fermentation with associated gas pressure. Further, although the bulking effect may induce satiety, the effect is often short-lived.

A well established principle for treating heartburn, where the acidic contents of the stomach reflux into the oesophagus, is to provide a divalent cation (typically calcium) gelled alginate or pectin barrier to prevent the reflux. One such commonly available heartburn therapy is 'Gaviscon'®. The production of such rafts/barriers with alginic acid or low methyl pectin has been discussed elsewhere (Jorgen et al, 1988; Foldager et al., 1993; Cox, 1996a and 1996b; Mandel et al., 2000; US patent application 20050063980).

### Glycogen storage disease

In the normal human, the anabolism and catabolism of glycogen is normally co-ordinated and regulated. The deposition of glycogen is promoted by insulin whilst the hydrolysis of glycogen and conversion to glucose is promoted by adrenaline (especially muscle) and glucagons (especially liver).

In glycogen storage disease (GSD) there is an inherited defect with respect to the deposition or hydrolysis of glycogen (http://www.agsd.org.uk/home/information.asp; http://agsdus.org/body_whatis_1.html) and consequently the concentration of blood glucose. Figure 1 outlines the principles of glycogen metabolism.

The most common types of glycogen storage disease are as follows:

In Type I (Von Gierke Disease) individuals suffer from a lack of glucose-6-phosphatase activity ('h' in Figure 1) and hence cannot generate glucose from glycogen. Consequently they need to be tube fed to maintain blood glucose.

In Type II (Pompe's Disease) individuals suffer from a lack of α-glucosidase activity ('i' in Figure 1). Infants often die of this form very young.

In Type III (Cori's Disease) individuals suffer from a lack of debranching enzyme activity ('i' in Figure 1). Treatment usually consists of a high protein diet.

In Type IV (Anderson's Disease) individuals suffer from a lack of branching enzyme activity ('e' in Figure 1). Liver transplantation is the only viable therapy.

In Type V (McArdle's Disease) individuals suffer from a lack of muscle phosphorylase activity ('f' in Figure 1). Extensive exercise should be avoided.

In Type VI (Her's Disease) individuals suffer from a lack of liver phosphorylase activity ('f' in Figure 1). There is a male X- chromosome link.

In Type VII (Tarui's Disease) individuals suffer from a lack of muscle phosphofructokinase activity. Extensive exercise should be avoided.

In Type IX individuals suffer from a lack of liver phosphorylase activity. There is a male X-chromosome link and it is comparable to type VI.

Low blood glucose can be treated by the slow administration of glucose (oral or intra-venous), or from starch hydrolysates (e.g. maltose, dextrins etc.) or from native starch where glucose is liberated as a consequence of digestion. In practice 'corn-starch', which is normal maize starch, is used to treat glycogen storage disease (especially during sleep) due to availability and to lack of a superior alternative in terms of digestive response. The starch must be slowly digested and not converted to glucose rapidly or excreted with little hydrolysis. In other clinical conditions (such as diabetes mellitus) there is also the need to supply glucose slowly and from a non-sugar based matrix (e.g. cakes, biscuits, sweets etc.). This can, therefore, also be achieved by starch (hydrolysis in the gut) and is important for night time regimes where glucose is essential in the blood but within a controlled form.

However, although many currently available starch preparations used in the treatment of conditions such as GSD have prolonged glucose release profiles compared to glucose and maltodextrin based products, the time period over which the products enable serum glucose levels to be maintained within an acceptable range is relatively short. However, there is a great need for alternative means of maintaining serum glucose levels within safe ranges over a longer period of time than that afforded by the conventional treatments.

### Summary of the Invention

The present inventors have surprisingly determined that the ingestion of a composition which produces a gastric raft in the stomach enables the induction of a feeling of satiety which, compared to known methods of inducing satiety, such as the ingestion of high fibre foodstuffs, is considerably prolonged. Moreover, the use of such gastric raft compositions for the induction of satiety may avoid the discomfort often felt by individuals when high fibre foodstuffs and food supplements are employed and may reduce the incidence and/or severity of any colonic disturbances which may occur as a side effect of such diets.

A gastric raft composition comprising:
a gel forming biopolymer and one or more processed starches, wherein said processed starch integrates with the gel-forming biopolymer in the formation of a raft on contacting the gastric raft composition with gastric acid.

Using the compositions of the invention, the inventors have found that satiety may be maintained for considerably longer time periods than with conventional compositions, for example the use of high fibre bulking agents.

In one embodiment of the invention, satiety may be maintained for greater than two hours, for example, greater than 3, 4, 5, or 6 hours.

Gastric raft compositions for use in the invention typically comprise gel forming biopolymers, which, on contact with stomach acid, form a gelatinous raft or foam which floats on the stomach contents. In order to produce a gel which floats, the compositions generally comprise a material, such as a calcium carbonate, which on contact with the gastric acid, produce non-toxic gas, for example carbon dioxide, which contributes to the buoyancy of the gel.

Any suitable biopolymer may be used in the invention. For example, the composition may comprise alginate, pectin, xanthan gum or a mixture thereof.

Any suitable gas forming agent may be used in the compositions of the invention. Such agents are typically bicarbonates of an alkali or alkaline earth metal, for example, sodium bicarbonate, potassium bicarbonate or calcium carbonate.

Moreover, in the course of their investigations, the inventors have surprisingly found that, by incorporating processed starches such as pregelatinised starches, acid thinned starches and/or dextrins, into the raft compositions in addition to gel-forming agents such as alginate and/or pectin, rafts of improved strength and integrity are produced. This may result in prolonged retention of the raft in the stomach of an individual who has ingested the gastric raft composition, may prolong satiety maintenance and/or appetite suppression effects and delay the delivery of any nutrients within the raft composition to sites of absorption in the small intestine.

Thus, the gastric raft composition of the invention, comprises a processed starch.

Indeed, in a further aspect of the invention, there is provided a gastric raft composition comprising:
one or more gel forming ingredient(s),
a gas forming agent and
one or more processed starches wherein said one or more processed starches integrates with the gel forming agent in the formation of a raft on contacting the gastric raft composition with gastric acid.

In a further aspect of the invention, there is provided a gastric raft composition according to the the invention for use in medicine.

Any suitable processed starches may be used in the compositions of and for use in the invention. For example, suitable processed starches include, but are not limited to, pregelatinised starch, acid thinned starch and/or dextrin. In contrast, unprocessed starches, i.e. native starches, are not appropriate for this purpose as they do not dissolve/disperse though the raft matrix and do not provide the functional advantages of processed starches.

In one embodiment of the gastric raft compositions of and for use in the invention, processed starch is present in sufficient quantities to form, in use, an active structural component of the raft. Thus, in one embodiment, the processed starch is present at a concentration of greater than 5%, for example greater than 10%, 20%, 30%, 40% w/w of the gastric raft composition. In one embodiments of the invention the ratio of starch to gel-forming agent, such as alginate, pectin or xanthan gum is in the range, for example in the range 1:6 to 1:3.

In an embodiment of the aspect of the invention in which starches are utilised in the gastric raft compositions, the starch is a solubilised starch derivative such as acid thinned starch or a dextrin. This has the advantage over pre-gelatinised starch in that it has less tendency to crystallise from solution (retrograde).

However, pre-gelatinised waxy (low amylose/high amylopectin) starches do not tend to retrograde quickly. Therefore, in an alternative embodiment the starches are pre-gelatinised waxy starches.

In a particular embodiment of the invention, dextrins may be used as a starch component of the gastric raft composition. A number of different maltodextrins (typically defined as dextrose equivalence (DE) 1-20) and beta-limit dextrin were used for this purpose. The dextrins formed strong rafts (especially for the beta-limit dextrin and maltodextrins with a DE of <10).

Whilst dextrins may be used in one embodiment of the invention, more extensively hydrolysed starches, for example glucose syrups may be used.

The discovery by the present inventors that the use of processed starches as active structural components of the raft composition increases the structural integrity of the raft and thus may enhance its effectiveness and may prolong its action in the stomach is not only of relevance to the use of such gastric raft compositions in the maintenance of satiety and the treatment of obesity but also the known uses of gastric raft compositions, for example, in the treatment of gastro-oesophageal acid reflux or heartburn.

In a further aspect of the invention, there is provided the use of a gastric raft composition according to the invention in the preparation of a medicament for the treatment of gastro-oesophageal reflux disease.

Such raft compositions may also be used as drug delivery means for targeting of drugs to the stomach. The use of processed starch in the raft compositions has the additional advantage in that it may form 'complexes' (e.g. amylose-lipid or amylose-drug or amylose iodine) and associations unique to starchy molecules and thus provide a delivery matrix which is different in design and physiological effect to rafts without processed starch molecules. The prolonged retention of the raft compositions of the invention in the stomach makes them particularly useful in the sustained release of active ingredients over a period of time, for example, 2 to 8 hours.

In a further aspect of the invention, there is provided the use of a gastric raft composition according to the invention, wherein said gastric raft composition comprises an active agent for treatment of a medical condition, in the preparation of a medicament for the treatment of a medical condition.

Due to their prolonged retention in the stomach, the gastric raft compositions of the invention are particularly useful in the treatment of gastric conditions. Thus, in one embodiment of the invention, the medical comditions is a gastric condition. For example, active agents which may be used in the gastric raft compositions of and for use in the invention include agents for the treatment of any gastric condition, such as gastritis, dyspepsia, peptic ulcer, gastric carcinoma, or infection such as Heliobacteria pylori infection.

The gastric raft compositions of and for use in the invention may also be used for sustained release of active agents. Thus, where applicable, the active ingredients may be provided in or adapted for sustained release.

An additional benefit of the gastric raft compositions of the invention is that such rafts can prolong retention of nutrient sources in the stomach. Such energy sources may include the processed starch components of the raft, and, optionally other energy sources such as lipids, proteins, vitamins, minerals and microorganisms, such as probiotic microorganisms if included in the raft compositions. By their retention in the stomach, the raft compostions of the invention reduce the rate at which the nutrients enter the small intestine and thus delay the digestion and absorption of such nutrients.

Thus, the rafts may be used as a means of controlling the rate of nutrient release from the stomach including energy derived from the processed raft's starch component and/or other nutrients (e.g. proteins, lipids, vitamins and minerals embedded in the matrix). These non-starch elements may form loose associations with the rafts or, as in the case of fatty acids, form true complexes with, for example an amylose fraction and hence modify progression through the stomach.

Hence, in a further aspect of the invention provides a gastric raft according to the invention for use in slowing the release of nutrients from the stomach to the small intestine.

Such nutrients may include, but are not limited to, one or more of proteins, lipids, vitamins and minerals, Encapsulated (probiotic) bacteria or associated prebiotic materials.

The discovery that gastric raft compositions of the invention may be used to delay nutrient release has implications beyond prolongation of satiety. Importantly, prolonged retention of nutrients in the stomach enables the use of the gastric raft compositions of the invention in the treatment of diseases associated with hypoglycaemia, such as glycogen storage disease.

In a further aspect of the invention, there is provided the use of a gastric raft composition according to the invention in the preparation of a medicament for controlling serum glucose levels in an individual.

In a further aspect, the invention provides the use of a gastric raft composition according to the invention in the preparation of a medicament for treating or preventing hypoglycaemia.

In a further aspect, the invention provides the use of a gastric raft composition according to the invention in the preparation of a medicament for treating or preventing a condition associated with hypoglycaemia.

In one embodiment of the invention, said treatment is treatment to prevent or decrease night-time hypoglycaemic episode(s).

By using raft compositions of the invention, the delivery of starches may be prolonged and thus treatments for conditions characterised by hypoglycaemic episodes may be improved. To further prolong the absorption of the starches, waxy starches may be used in the raft compositions as sources of α-glucan, thus enabling significant improvement to control over the rate of glucose formation and appearance in the blood mammals. Such starches significantly outperform the conventionally used 'corn starch' (native maize starch) in terms of duration of glucose release due to amylase hydrolysis in the small intestine.

Moreover, the inventors have shown that the glucose release profile may be further dramatically prolonged by modifications to the processed starch e.g. by hydrothermal treatment for example, by heat moisture treatment. Indeed, hydrothermal treatment also provides considerable improvement in conventional non-waxy starches. Thus, waxy starch can be substituted by any hydrothermally treated starch, preferably heat moisture treated starch (whether waxy or non-waxy).

The compositions of the invention may be used to treat individuals with any disease associated with the presence or susceptibility to hypoglycaemia. Such diseases include, but are not limited to diabetes (Type I or Type II), glycogen storage disease, liver disease, for example, liver cirrhosis.

With many dietary regimes employed as a means of losing weight, a side effect may often be halitosis.

This may be due to, for example, the breakdown of ketones in the body, giving rise to "ketotic breath".

The present inventors have found that, in addition to providing a satiety inducing effect, gastric raft compositions also provide an effective means of treating halitosis.

Also provided by the present invention is the use of a gastric raft composition in the preparation of a medicament for the treatment of halitosis.

Preferred features of each aspect of the invention are as for each of the other aspects mutatis mutandis.

### Detailed description

As described above, the present inventors have determined a novel means of inducing and maintaining satiety /controlling the release of nutrients and drugs from the stomach using gastric raft compositions. As described above, the discovery that gastric raft compositions enable a prolonged feeling of satiety (and delay nutrient release) make these compositions particularly useful in dietary regimes, for example in the treatment of obesity. The use of such compositions has a number of advantages over conventional treatments.

Firstly, conventional dietary agents such as fibre rich food supplements or meal replacements merely act by a bulking effect in the stomach, the effect passing as the foods pass from the stomach. In contrast, because the gastric raft compositions float on the gastric contents, the raft is maintained in the stomach for a considerably longer time than conventional high fibre dietary supplements.

Secondly, with conventional fibre rich dietary foods, a relatively large volume of bulking polysaccharide is required for the satiety inducing effect. This may result in colonic pain as a result of colonic fermentation. In contrast, using gastric raft compositions, the amount of polysaccharides, such as alginate or pectin is considerably less. As a result, colonic pain as a result of colonic fermentation should be reduced/ not occur.

Thirdly, a potential side effect of conventional fibre rich dietary food supplements comprising polysaccharides such as alginates and pectins in large concentrations is demineralisation of the body as a result of cation binding to the polysaccharide. As gastric raft compositions comprise only small quantities of such polysaccharides, demineralisation side effects can be reduced. Furthermore, in those embodiments of the invention in which processed starches, which do not contain cation binding groups, are used, the amount of alginates /pectins and thus the amount of cation binding groups can be further reduced, thus further reducing potential side effects.

Fourthly, the gastric raft composition of and for use in the invention has a satiety inducing effect which is purely physical, which does not rely on the provision of any pharmacological agent, thus enabling the avoidance of any pharmacological side effects or interactions, which may be encountered when using appetite suppressing drugs. Of course, although the gastric rafts of and for use in the present invention do not require the provision of pharmacological agents to produce a satiety inducing effect, in one embodiment the gastric rafts of and for use in the invention be comprise or be formulated together with pharmacological agents, which may have a satiety inducing and/or appetite suppressing effect.

### Gastric Rafts

As described above, the inventors have shown that gastric raft compositions may be used to provide satiety inducing effects and thus may be used in the treatment of obesity.

In the context of the present invention, a gastric raft composition is a preparation which, on contact with gastric acid, forms a gelatinous foam or raft which floats on the stomach contents.

Any suitable gastric raft composition may be used in the invention. Gastric raft compositions typically comprise a gel forming agent, usually a biopolymer, and a material capable of producing gas, typically carbon dioxide, when contacted with gastric acid.

Biopolymers which may be used as the gel forming agent include (but not exclusively) alginates, pectins and xanthan gum, or combinations thereof.

In one embodiment, the gel-forming agent is an alginate. Alginic acid is a naturally occurring polysaccharide obtained (predominantly) from the various species of brown seaweed (*Phaeophyceae*). It is a linear molecule consisting mainly of residues of β-(1,4)-linked D-mannuronic acid and α-(1,4)-linked L-guluronic acid. Alginic acid contains at least three different types of polymer segments: poly β-D-mannuronic acid segments, poly α-L-guluronic acid segments, and segments with alternating sugar units. The ratios of the constituent monomers and the nature of the chain segments vary with the source and determine the specific properties of the polysaccharide. A useful property of alginates is their ability to form gels by reactions with cations, especially divalent cations such as calcium ions. The type of gel formed depends on the source of alginic acid.

Alginates with a high percentage of polyguluronate segments from more strong, rigid and brittle calcium-alginate gels whereas alginates with a higher percentage of polymannuronate segments form soft, more elastic and deformable gels. The rate of gel formation as well as the quality and texture of the resultant gel can be controlled by the solubility and availability of the cation source.

In one embodiment, alginic acids having a ratio of guluronic to mannuronic acid in the range 70:30 to 20:80, for example 40:60 may be used for the present application. In addition alginic acids used may typically (but not exclusively) contain between 15 and 70% of poly (β-D-mannuronic acid) segments; between 15 and 60% of poly (α-L-guluronic acid) segments and between 15 and 40% of segments with alternating sugar units.

In one embodiment, the gel-forming agent is pectin. The pectin(s) may, for example, be derived from citrus fruits.

If pectins are used these may be selected from (but not exclusively), for example, one or more of polygalacturonic acid and de-esterified or partially de-esterified pectins or derivatives thereof. Polygalacturonic acid is an essentially linear molecule. Pectins having a typical molecular weight in the range 10,000 to 70,000, for example 20,000 to 60,000 , such as 25,000 to 50,000, may be used. As with the alginic acid, the pectins may be used alone or in combination with other polysaccharides that gel in the presence of a divalent or multivalent cation.

For production of the gas, carbonates or bicarbonates of an alkali or alkaline metal, for example sodium, potassium or calcium may be used. Particular examples of salts which may be used include calcium carbonate and sodium hydrogen carbonate.

In use, the 'gastric rafts' are generated from the gel forming agent, for example alginate or pectin, which may be more or less gelled by the presence of cationic salts (e.g. calcium) floating on a bed of carbon dioxide - such ingredients are not dispersed as 'thickeners' throughout the stomach.

Rafts are distinct from using polysaccharides (alone or with adjuncts) to bulk the stomach. The generation of the gas phase from the ingested composition (solid or liquid) is responsible for this floating effect. The raft may be retained in the stomach for a prolonged period of time, for example more than 3 hours, such as more than 4, 5, 6, 7 or 8 hours. Rafts thus provide a much longer retention of polysaccharide within the stomach. Moreover, using raft compositions, the amount of polysaccharide required is considerably less than that required for satiety inducing agents which act through a mere bulking effect.

In one embodiment of the gastric raft composition of and for use in the invention, the composition comprises per unit dose less than 2g, preferably less than 1g of gel forming ingredient(s). For example, the composition may comprise less than 0.5g of gel forming ingredient(s).

### Starches

As described above, the inventors have surprisingly shown that the integrity of the raft composition may be improved by the incorporation of processed starches and/or starch derivatives as structural components of the raft.

Furthermore, there are a number of additional potential advantages associated with the use of starches in the gastric raft composition. For example, as starches are considerably cheaper than alginates/pectins, the cost of producing the gastric raft composition may be reduced compared to conventional raft compositions. The use of starches in the raft compositions also provides improved interactions of the starch fraction with drugs and nutrients (e.g. complexes regulating delivery which cannot be achieved with pectins or alginates).

Other benefits of using processed starches as structural components of the raft compositions include: improved raft strength with associated positive viscosity features; moderation of mineral binding; provision of some calories to facilitate certain vitamin utilisation; reduction in glycaemic index (GI) response of the processed starch due to delayed release of calories (sugars) into the intestine and blood; reduction of viscosity of the matrix through the intestine as the starch fraction is hydrolysed by amylases; ease of swallowing; reduction of fermentable bulk which may potentially cause colonic discomfort and flatulence.

Accordingly, in preferred embodiments of the invention, the gastric raft composition comprises one or more processed starches or starch derivatives.

In the present invention, any suitable processed starch or starch hydrolysate may be used (e.g. gelatinised, pre-gelatinised, acid thinned, dextrins, enzyme treated, fermented etc.). Native starches are insoluble and hence do not disperse and solubilise within the raft matrix.

In one embodiment, the starch of and for use in the invention is dextrin. Note that maltodextrins may be more extensively hydrolysed to glucose syrups. This application does not exclude any solubilised starch fractions.

Comparisons between different starches are shown in the Table below.

**Table 1. Some properties of starches and starch fractions in rafts**

| | **Native starches** | **Gelatinised/ amorphous starch** | **Hydrolysed/ depolymerised starch** |
|---|---|---|---|
| **Solubility** | Insoluble | Soluble | Very soluble |
| **Molecular interactions dispersion with alginate/ pectin in solution/gel** | No | Yes | Yes |
| **Digestible by man** | No | Yes | Yes |
| **Interact easily with other molecules** | No | Yes | Yes |
| **Complexing ease** | No | Yes | Yes |
| **Gel forming** | No | Yes | Yes |
| **Discrete phase** | Yes | No | No |

In a particularly preferred embodiment of the gastric raft composition of and for use in the invention, the starch is present in the gastric raft composition in a form and at a concentration sufficient to enable starch molecules to become incorporated within the raft, when the raft is formed by contact of the gastric raft composition with gastric acid. In preferred embodiments, the starch, when present, is present at a concentration such that the ratio of starch to gel forming agent in the gastric raft composition, is in the range 5-25% w/w of the polysaccharide.

### Starch Hydrolysates

Starches can be solubilised by treating with acids and enzymes (amylases and amyloglucosidases). Commercial maltodextrins (below) are usually defined as up to DP 20 beyond which they are defined as glucose syrups.

### Dextrins

If starches are hydrolysed (typically chemically with acids or enzymatically with α-amylase and amyloglucosidase) smaller molecules called 'dextrins' are generated. Products may be as small as the smallest possible monosaccharide glucose or be slightly hydrolysed but still oligo/polymeric. Glucose syrups are made from starch hydrolysis and contain variable proportions of sugars and dextrins depending on the nature and extent of conversion.

Maltodextrins are DP20 or less, GRAS quality, tasteless and very soluble. They are easily digestible and are used in energy drinks because of their solubility and reportedly relatively slow digestibility compared to glucose (which is simply absorbed). The difference in rate of glucose appearance in the blood as a consequence of drinking glucose or maltodextrin solutions is relatively small (e.g. -45minutes) because of the extent of conversion of the maltodextrin.

### Hydrothermally Treated Starch

As discussed above and shown in the examples below, the inventors have found that particularly good results are obtained when using hydrothermally treated starch.

Two main methods are currently used for the hydrothermal treatment of starch: heat-moisture treatment (high temperature, low moisture) and annealing (high moisture, low temperature).

### Heat Moisture Treated Starch (HMT Starch)

Heat and moisture treated starch is typically produced by exposing moist starch (e.g. 15-30% moisture) to temperatures of e.g. 95°C to 130° for periods up to 30 hours (typically 16-24). These ranges do not exclude other heat-moisture profiles. For example, HMT starch for use in the invention may be produced by thermally treating starch in a sealed container under the following conditions: 20% moisture and 105°C for 16 hours. The treated starch may then be cooled to room temperature, air-dried and then passed through 300um sieve.

Such heat moisture treatment results in a number of significant property changes to starches. The extent of the effect varies with the type of starch but in general the effects are:
- increased gelatinisation temperature
- reduced water absorption and swelling power
- changed X-ray diffraction pattern
- increased enzyme susceptibility

As described herein, although heat moisture treatment results in starches having increased susceptibility to enzymatic degradation, the inventors have shown that when used in methods of the invention, heat moisture treated starches provide significantly greater prolongation of the time period over which serum glucose levels are maintained compared to the corresponding non heat moisture treated starches. Accordingly, in one embodiment of the gastric raft composition of and for use in the invention, the gastric raft composition comprises a heat moisture treated starch.

### Treatment/Therapy

"Treatment" (which, unless the context demands otherwise, is used interchangeably with "therapy", includes any regime that can benefit a human or non-human animal. The treatment may be in respect of an existing condition or may be prophylactic (preventative treatment). Treatment may include curative, alleviation or prophylactic effects.

### Pharmaceutical Formulations

The gastric raft compositions of and for use in the present invention will typically be provided as a nutritional/clinical nutritional or pharmaceutical composition, which will generally comprise a suitable pharmaceutical excipient, diluent or carrier, suitable for oral administration.

Compositions for oral administration may be in for example tablet, capsule, powder or liquid form. The liquid form may be flavoured and sweetened and contain other components to make it more acceptable as a drink type product. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. The key components may also be incorporated into food and feed systems such as drinks, soups and meals where the components may still interact to form the raft systems. Thus, the gastric raft compositions of and for use in the invention may be provided in food compositions.

The invention extends to a therapeutic food composition for the treatment of diseases characterised by hypoglycaemic episodes, wherein said composition comprises a gastric raft composition according to the fourth aspect of the invention.

The invention further extends to a therapeutic food composition for the treatment of obesity, wherein said composition comprises a gastric raft composition according to the fourth aspect of the invention.

The food compositions of and for use in the present invention may consist solely of said gastric raft compositions or may comprise further additives. Such additives may contribute merely to the palatability of the composition, e.g. flavourings, or may contribute significant calorific value, for example, sugars with a more rapid release profile than the starches, or lipids. These compounds may be incorporated to slow gastric emptying and facilitate the effect (e.g. amino acids, lipids etc.).

The therapeutic food composition can take a variety of forms, for example as a food, a food supplement, a liquid, an emulsion or mixture thereof. It may be prepared as a ready to eat foodstuff, for example as a snackbar, a baked product, pasta or drink.

The invention will now be described further in the following non-limiting examples.

### Example 1

Liquid versions of alginic acid were made as follows:
Samples (0.7g or 0.5g) of sodium alginate (Manugel LBA, International Specialty Products, Koln), 0.2g calcium carbonate (12467, Acros Organics, Geel) and 0.25g sodium hydrogen carbonate (144-55-8, Aldrich, Gillingham) were dissolved in 10 ml distilled water. In addition, to some liquids flavouring and/or colouring was added.

These were poured onto 20ml 1M HCl contained in 100ml beakers as described in Table 1. The rafts that were produced functioned well.

The mixture was also consumed by up to ten volunteers and the feeling of satiety was described by the volunteers. The sensation of satiety lasted for up to around six hours. The individuals did not crave snacking between meals and hence there were distinct reductions in calorie consumption.

### Example 2

Liquid versions of low methyl pectin were made as follows:
Samples (0.7g or 0.5g) of low methyl pectin (LM-104, CP Kelco, Lille/Svensked), 0.2g calcium carbonate (12467, Acros Organics, Gee1) and 0.25g sodium hydrogen carbonate (144-55-8, Aldrich, Gillingham) were dissolved in 10 ml distilled water. In addition to some liquids, flavouring and/or colouring was added.

These were poured onto 20ml 1M HCl contained in 100ml beakers as described in Table 1. The rafts that were produced functioned well.

The mixture was also consumed by up to ten volunteers and the feeling of satiety was described by the volunteers. The sensation of satiety for most individuals lasted for up to around six hours. The individuals did not crave snacking between meals and hence there were distinct reductions in calorie consumption.

Mixtures of alginate and pectin, as the polysaccharide fraction were also tested and were found to perform well.

### Example 3

Samples (0.5g) of sodium alginate (Manugel LBA, International Specialty Products, Koln), 0.2g pregelatinised starch (NSG4, National Starch and Chemical, Manchester), 0.2g calcium carbonate (12467, Acros Organics, Gee1) and 0.25g sodium hydrogen carbonate (144-55-8, Aldrich, Gillingham) were dissolved in 10 ml distilled water. In addition to some liquids, flavouring and/or colouring was added.

These were poured onto 20ml 1M HCl contained in 100ml beakers as described in Table 1. The rafts that were produced functioned well.

The mixture was also consumed by up to ten volunteers and the feeling of satiety was described by the volunteers. The sensation of satiety lasted for up to around six hours. The individuals did not crave snacking between meals and hence there were distinct reductions in calorie consumption. The cost of product was cheaper than for pure alginate or pectin (as the polysaccharide) in view of the dilution by starch.

### Example 4

Samples (0.5g) of sodium alginate (Manugel LBA, International Specialty Products, Koln), 0.2g dextrin (Crystal Tex 626, National Starch and Chemical, Manchester), 0.2g calcium carbonate (12467, Acros Organics, Gee1) and 0.25g sodium hydrogen carbonate (144-55-8, Aldrich, Gillingham) were dissolved in 10 ml distilled water. In addition to some liquids, flavouring and/or colouring was added.

These were poured onto 20ml 1M HCl contained in 100ml beakers as described in Table 1. The rafts that were produced functioned well.

The mixture was also consumed by up to ten volunteers and the feeling of satiety was described by the volunteers. Once again, the sensation of satiety lasted for up to around six hours. The individuals did not crave snacking between meals and hence there were distinct reductions in calorie consumption.

### Example 5

Samples (0.5g) of low methyl pectin (LM-104, CP Kelco, Lille Svensked), 0.2g pregelatinised starch (NSG4, National Starch and Chemical, Manchester), 0.2g calcium carbonate (12467, Acros Organics, Geel) and 0.25g sodium hydrogen carbonate (144-55-8, Aldrich, Gillingham) were dissolved in 10ml distilled water. In addition to some liquids, flavouring and/or colouring was added.

These were poured onto 20ml 1M HCl contained in 100ml beakers as described in Table 1. The rafts that were produced functioned well.

The mixture was also consumed by up to ten volunteers and the feeling of satiety was described by the volunteers. The sensation of satiety lasted for up to around six hours. The individuals did not crave snacking between meals and hence there were distinct reductions in calorie consumption. The cost of product was cheaper than for pure alginate or pectin (as the polysaccharide) in view of the dilution by starch.

### Example 6

Samples (0.5g) of low methyl pectin (LM-104, CP Kelco, Lille Svensked), 0.2g dextrin (Crystal Tex 626, National Starch and Chemical, Manchester), 0.2g calcium carbonate (12467, Acros Organics, Geel) and 0.25g sodium hydrogen carbonate (144-55-8, Aldrich, Gillingham) were dissolved in 10ml distilled water. In addition to some liquids, flavourings and/or colouring was added.

These were poured onto 20ml HCl contained in 100ml beakers as described in Table 1. The rafts that were produced functioned well.

The mixture was also consumed by up to ten volunteers and the feeling of satiety was described by the volunteers. Once again, the sensation of satiety lasted for up to around six hours. The individuals did not crave snacking between meals and hence there were distinct reductions in calorie consumption.

### Example 7

Samples (500mg/700mg) of sodium alginate (Manugel LBA, International Specialty Products, Koln), 200mg calcium carbonate (12467, Acros Organics, Geel) and 250mg sodium hydrogen carbonate (144-55-8, Aldrich, Gillingham) were blended together and appropriately sized samples packed into gelatine or HPMC capsules. In addition to some blends, flavour and/or colour was added.

In the laboratory, the capsules or the capsule contents were dropped onto 20ml of 1M HCl within 100ml beakers (directly). The rafts were less well formed than for the liquid version of the technology. It was assumed, therefore, that some hydration was desirable before swallowing and that chewing powder/dry dosage forms prior to swallowing might be useful. Alternatively, the powder (tablet/capsule etc.) could be dissolved in water prior to swallowing. The material is clearly more appropriate to transport in a dry form.

A capsule was also consumed by up to ten volunteers after or without eating and the feeling of satiety was described by the volunteers.

### Example 8

Samples (500mg) of sodium alginate (Manugel LBA, International Specialty Products, Koln), 200mg pregelatinised starch (NSG4, National Starch and Chemical, Manchester) or dextrin (Crystal Tex 626, National Starch and Chemical, Manchester), 200mg calcium carbonate (12467, Acros Organics, Geel) and 250mg sodium hydrogen carbonate (144-55-8, Aldrich, Gillingham) were blended together and appropriately sized samples packed into gelatine or HPMC capsules. In addition to some blends, flavour and/or colour was added.

In the laboratory, the capsules or the capsule contents were dropped onto 20ml of 1M HCl within 100ml beakers (directly). The rafts were less well formed than for the liquid version of the technology (although better than for pure alginate or pectin alone). It was assumed, therefore, that some hydration was desirable before swallowing and that chewing powder/dry dosage forms prior to swallowing might be useful. Alternatively, the powder (tablet/capsule etc.) could be dissolved in water prior to swallowing. The material is clearly more appropriate to transport in a dry form.

A capsule was also consumed by up to ten volunteers after or without eating and the feeling of satiety was described by the volunteers.

It is recognised that a number of formats of the technology may be developed for the control of satiety and related applications.

### Example 9

### Mechanical properties of rafts

The following compositions (liquid) of rafts were formulated: sodium alginate (Manugel LBA, International Specialty Products, Koln), pectin (LM-104, CP Kelco, Lille/Skensved), alginate-pregelatinised starch (NSG4, National Starch and Chemical, Manchester), alginate-dextrin (Crystal Tex 626, National Starch and Chemical, Manchester), pectin-pre-gelatinised starch or pectin-dextrin rafts. The mechanical properties of the rafts produced from the compositions were tested *in vitro* where the liquid composition (raft) was poured onto 20ml of 1M HCl in 100ml screw topped flasks at room temperature. The results are summarised in Table 2.

Pectin based rafts were apparently more robust than alginate based rafts.

It was apparent that the presence of the starch/starch derivatives surprisingly made more durable and effective raft systems than alginate or pectin alone. The rafts have a much greater integrity when poured onto HCl (to represent the stomach) as described

Table 2: Gastric raft compositions comprising sodium alginate (Manugel LBA, International Specialty Products, Koln), pectin (LM-104, CP Kelco, Lille/Skensved), alginate-pre-gelatinised starch (NSG4, National Starch and Chemical, Manchester), alginate-dextrin (Crystal Tex 626, National Starch and Chemical, Manchester), pectin-pre-gelatinised starch or pectin-dextrin were tested *in vitro.*

**Table 2 (part i)**

| **Raft attribute** | **Sodium Alginate (0.5g or 0.7g dissolved in 10ml distilled water) as sole carbohydrate** | **Pectin (0.5g or 0.7g dissolved in 10ml distilled water) as sole carbohydrate** | **Pectin (0.5g) plus dextrin (0.2g) dissolved in 10ml distilled water** |
|---|---|---|---|
| Cohesiveness | Good | Good | Very good |
| Mechanical strength | Good | Good | Very good |
| Relative cost | Relatively high | Relatively high | Cheaper than pure pectin |
| Colour | White | White | White |
| Potential digestibility | Indigestible | Indigestible | Partially digestible |
| Proposed use for satiety | Yes | Yes | Yes |
| Proposed use for heartburn | Yes | Yes | Yes |
| Proposed use for halitosis | Yes | Yes | Yes |
| Proposed use for stomach therapy | Yes | Yes | Yes |

**Table 2 (part ii)**

| **Raft attribute** | **Sodium Alginate (0.5g or 0.7g dissolved in 10ml distilled water) as sole carbohydrate** | **Pectin (0.5g or 0.7g dissolved in 10ml distilled water) as sole carbohydrate** | **Pectin (0.5g) plus pre-gelatinised starch (0.2g) dissolved in 10ml distilled water** |
|---|---|---|---|
| Cohesiveness | Good | Good | Very good |
| Mechanical strength | Good | Good | Very good |
| Relative cost | Relatively high | Relatively high | Cheaper than pure pectin |
| Colour | White | White | White |
| Potential digestibility | Indigestible | Indigestible | Partially digestible |
| Proposed use for satiety | Yes | Yes | Yes |
| Proposed use for heartburn | Yes | Yes | Yes |
| Proposed use for halitosis | Yes | Yes | Yes |
| Proposed use for stomach therapy | Yes | Yes | Yes |

**Table 2 (part iii) :**

| **Raft attribute** | **Sodium alginate (0.5g or 0.7g) dissolved in 10ml distilled water** | **Pectin (0.5g or 0.7g) dissolved in 10ml distilled water** | **Alginate (0.5g) plus pre-gelatinised starch (0.2g) dissolved in 10ml distilled water** |
|---|---|---|---|
| Sample (10ml) poured onto 20ml 1M HCl and left statically | Up to 6 hours | > 8 hours | Up to 6 hours |
| Sample (10ml) poured onto 20ml 1M HCl and agitated by magnetic stirrer at 22ºC for 8 hours | Up to 2 hours | Up to 8 hours | Up to 2 hours |
| Sample (10ml) poured onto 20ml 1M HCl and agitated by shaking water bath (-30 cycles minute) at 22ºC for 8 hours | Up to 2 hours | > 8 hours | Up to 3 hours |

**Table 2 (part iv):**

| **Raft attribute** | **Alginate (0.5g) plus dextrin (0.2g) dissolved in 10ml distilled water** | **Pectin (0.5g) plus pre-gelatinised starch (0.2g) dissolved in 10ml distilled water** | **Pectin (0.5g) plus dextrin (0.2g) dissolved in 10ml distilled water** |
|---|---|---|---|
| Sample (10ml) poured onto 20ml 1M HCl and left statically | >8 hours | > 8 hours | >8 hours |
| Sample (10ml) poured onto 20ml 1M HCl and agitated by magnetic stirrer at 22ºC for 8 hours | Up to 3 hours | > 8 hours | > 8 hours |
| Sample (10ml) poured onto 20ml 1M HCl and agitated by shaking water bath (-30 cycles minute) at 22ºC for 8 hours | Up to 2 hours | > 8 hours | > 8 hours |

### References

Di Lorenzo, C., Williams, C. M., Hajnal, F. and Valenzuela, J. E. (1988) Pectin delays gastric emptying and increases satiety in obese subjects. Gastroenterology 95, 1211-1215.
Foldager, J., Toftkjaer, H. and Kjoernaes, K. (1993) DK22993.
http://www.gaviscon.com
Hoad et al, Journal of Nutrition 134, 2293-2300.
Jorgen et al (1988) EP0286085.
Mandel et al, Alimentary Pharmacology and Therapeutics 14, 669-690.
Tiwary et al Journal of American College of Nutrition 16, 423-428.
Torsdottir et al (1991) Journal of Nutrition 121, 795-799.

## Claims

1. A gastric raft composition comprising:
a gel forming biopolymer and one or more processed starches, wherein said processed starch integrates with the gel-forming biopolymer in the formation of a raft on contacting the gastric raft composition with gastric acid.

2. The composition according to claim 1, wherein the ratio of processed starch to gel forming biopolymer is in the range 1:6 to 1:3.

3. The composition according to claim 1 or claim 2, wherein said one or more starches includes pre-gelatinised starch.

4. The composition according to claims 1 to 3, wherein said one or more starches includes dextrin.

5. The composition according to claims 1 to 4, wherein said polysaccharide gel-forming biopolymer comprises at least one of alginate, pectin or xanthan gum.

6. A composition according to any one of claims 1 to 5 for use in medicine.

7. A gastric raft composition according to any one of claims 1 to 5 for use in controlling serum glucose levels in an individual.

8. Use of a gastric raft composition according to any one of claims 1 to 5 in the preparation of a medicament for use in the treatment of gastroesophageal reflux disease.

9. Use of a gastric raft composition according to any one of claims 1 to 5 in the preparation of a medicament for treating or preventing hypoglycaemia.

10. Use of a gastric raft composition according to any one of claims 1 to 5 in the preparation of a medicament for treating or preventing diabetes (Type I or Type II), glycogen storage disease, or liver disease.

11. Use of a gastric raft composition according to any one of claims 1 to 5 in the preparation of a medicament for the induction of satiety.

12. Use of a gastric raft composition according to any one of claims 1 to 5 in the preparation of a medicament for suppression of appetite.

13. Use of a gastric raft composition according to any one of claims 1 to 5 in the preparation of a medicament for the treatment of obesity.

14. Use of a gastric raft composition according to any one of claims 1 to 5 in the preparation of a medicament for the treatment of halitosis.

15. The use according to any one of claims 7 to 14, wherein said one or more processed starches comprise hydrolysated starch.

16. The use according to any one of claims 7 to 15 wherein said gastric raft composition comprises per unit dose less than 0.5 g of gel forming biopolymer.

17. The use according to any one of claims 7 to 16, wherein, in use in the stomach of an individual, the gastric raft formed by the gastric raft composition is maintained in the stomach for at least two hours.

18. The use according to claim 17, wherein the gastric raft formed by the gastric raft composition is maintained in the stomach for at least four hours.

## Patentansprüche

1. Magen-Raft-Zusammensetzung, umfassend:
ein Gel-bildendes Biopolymer und eine oder mehrere verarbeitete Stärken, wobei sich die genannte verarbeitete Stärke mit dem Gel-bildenden Biopolymer bei der Bildung eines Rafts integriert, wenn die Magen-Raft-Zusammensetzung die Magensäure kontaktiert.

2. Zusammensetzung nach Anspruch 1, wobei das Verhältnis der verarbeiteten Stärken zum Gel-bildenden Biopolymer im Bereich von 1:6 bis 1:3 liegt.

3. Zusammensetzung nach Anspruch 1 oder Anspruch 2, wobei die eine oder mehreren Stärken eine vorgelierte Stärke enthalten.

4. Zusammensetzung nach den Ansprüchen 1 bis 3, wobei die eine oder mehreren Stärken Dextrin enthalten.

5. Zusammensetzung nach den Ansprüchen 1 bis 4, wobei das genannte Polysaccharid Gelbildende Biopolymer wenigstens eins von Alginat, Pektin oder Xanthangummi umfasst.

6. Zusammensetzung nach einem der Ansprüche 1 bis 5 zur medizinischen Verwendung.

7. Magen-Raft-Zusammensetzung nach einem der Ansprüche 1 bis 5 zur Verwendung der Regelung der Serumglukosewerte in einer Person.

8. Verwendung einer Magen-Raft-Zusammensetzung nach einem der Ansprüche 1 bis 5 zu Herstellung eines Medikaments zur Verwendung bei der Behandlung einer gastroösophagealen Reflux-Erkrankung.

9. Verwendung einer Magen-Raft-Zusammensetzung nach einem der Ansprüche 1 bis 5 zur Herstellung eines Medikaments für die Behandlung oder Verhütung von Hypoglykämie.

10. Verwendung einer Magen-Raft-Zusammensetzung nach einem der Ansprüche 1 bis 5 zur Herstellung eines Medikaments für die Behandlung oder Verhütung von Diabetes (Typ I oder Typ II), Glykogenspeicherkrankheit oder Leberkrankheit.

11. Verwendung einer Magen-Raft-Zusammensetzung nach einem der Ansprüche 1 bis 5 zur Herstellung eines Medikaments zum Induzieren eines Sättigungsgefühls.

12. Verwendung einer Magen-Raft-Zusammensetzung nach einem der Ansprüche 1 bis 5 zur Herstellung eines Medikaments zur Unterdrückung des Appetits.

13. Verwendung einer Magen-Raft-Zusammensetzung nach einem der Ansprüche 1 bis 5 zur Herstellung eines Medikaments zur Behandlung von Fettleibigkeit.

14. Verwendung einer Magen-Raft-Zusammensetzung nach einem der Ansprüche 1 bis 5 zur Herstellung eines Medikaments zur Behandlung von Mundgeruch.

15. Verwendung nach einem der Ansprüche 7 bis 14, wobei eine oder mehrere verarbeitete Stärken hydrolysierte Stärke umfasst.

16. Verwendung nach einem der Ansprüche 7 bis 15, wobei die genannte Magen-Raft-Zusammensetzung per Einheitsdosis weniger als 0,5 g des Gel-bildenden Biopolymers umfasst.

17. Verwendung nach einem der Ansprüche 7 bis 16, wobei bei der Verwendung im Magen einer Person das durch die Magen-Raft-Zusammensetzung gebildete Magen-Raft wenigstens zwei Stunden lang im Magen gehalten wird.

18. Verwendung nach Anspruch 17, wobei das durch die Magen-Raft-Zusammensetzung gebildete Magen-Raft wenigstens vier Stunden lang im Magen gehalten wird.

## Revendications

1. Composition de radeau gastrique comprenant :
un biopolymère formant un gel et un ou plusieurs amidons traités, lesdits amidons traités s'intégrant avec le biopolymère formant un gel dans la formation d'un radeau lors du contact de la composition de radeau gastrique avec l'acide gastrique.

2. Composition selon la revendication 1, dans laquelle le rapport de l'amidon traité au biopolymère formant un gel est compris dans la plage de 1/6 à 1/3.

3. Composition selon la revendication 1 ou la revendication 2, dans laquelle ledit ou lesdits amidons comprennent de l'amidon pré-gélatinisé.

4. Composition selon les revendications 1 à 3, dans laquelle ledit ou lesdits amidons comprennent une dextrine.

5. Composition selon les revendications 1 à 4, dans laquelle ledit biopolymère formant un gel polysaccharidique comprend au moins un composé parmi l'alginate, la pectine ou la gomme de xanthane.

6. Composition selon l'une quelconque des revendications 1 à 5, destinée à être utilisée en médecine.

7. Composition de radeau gastrique selon l'une quelconque des revendications 1 à 5, destinée à être utilisée dans la régulation des teneurs en glucose sérique chez un individu.

8. Utilisation d'une composition de radeau gastrique selon l'une quelconque des revendications 1 à 5, dans la préparation d'un médicament destiné à être utilisé dans le traitement d'une maladie de reflux gastro-oesophagique.

9. Utilisation d'une composition de radeau gastrique selon l'une quelconque des revendications 1 à 5, dans la préparation d'un médicament pour le traitement ou la prévention de l'hypoglycémie.

10. Utilisation d'une composition de radeau gastrique selon l'une quelconque des revendications 1 à 5, dans la préparation d'un médicament pour le traitement ou la prévention du diabète (Type I ou Type II), d'une glycogénose ou d'une maladie hépatique.

11. Utilisation d'une composition de radeau gastrique selon l'une quelconque des revendications 1 à 5, dans la préparation d'un médicament pour l'induction de la satiété.

12. Utilisation d'une composition de radeau gastrique selon l'une quelconque des revendications 1 à 5, dans la préparation d'un médicament pour la suppression de l'appétit.

13. Utilisation d'une composition de radeau gastrique selon l'une quelconque des revendications 1 à 5, dans la préparation d'un médicament pour le traitement de l'obésité.

14. Utilisation d'une composition de radeau gastrique selon l'une quelconque des revendications 1 à 5, dans la préparation d'un médicament pour le traitement de l'halitose.

15. Utilisation selon l'une quelconque des revendications 7 à 14, dans laquelle ledit ou lesdits amidons traités comprennent de l'amidon hydrolysé.

16. Utilisation selon l'une quelconque des revendications 7 à 15, dans laquelle ladite composition de radeau gastrique comprend par dose unitaire moins de 0,5 g de biopolymère formant un gel.

17. Utilisation selon l'une quelconque des revendications 7 à 16, dans laquelle, lors de l'utilisation dans l'estomac d'un individu, le radeau gastrique formé par la composition de radeau gastrique est maintenu dans l'estomac pendant au moins deux heures.

18. Utilisation selon la revendication 17, dans laquelle le radeau gastrique formé par la composition de radeau gastrique est maintenu dans l'estomac pendant au moins quatre heures.
